# EUROPEAN PATENT SPECIFICATION

(11) **EP 2 757 158 B1**
(45) Date of publication and mention of the grant of the patent: **04.09.2019**
(21) Application number: 11872406.1
(22) Date of filing: 14.09.2011
(51) Int. Cl.: C12P 7/64, C12N 9/18, C12N 11/14, C10M 101/04

(54) **ENZYMATIC PROCESS FOR THE SYNTHESIS OF ESTOLIDES**
ENZYMATISCHES VERFAHREN ZUR HERSTELLUNG VON ESTOLIDEN
PROCÉDÉ ENZYMATIQUE POUR LA SYNTHÈSE D'ESTOLIDES

(43) Date of publication of application: 23.07.2014
(73) Proprietor: Petroleo Brasileiro S.A. - PETROBRAS, CEP 20035-900 - Rio de Janeiro, RJ (BR)
(72) Inventor: GONÇALVES AGUIEIRAS, Érika Cristina, CEP: 20510-080 Rio de Janeiro, RJ (BR); DE OLIVEIRA VELOSO, Cláudia, CEP: 20520-050 Rio de Janeiro, RJ (BR); DE OLIVEIRA ROSAS, Danielle, CEP: 21320-120 Rio de Janeiro - RJ (BR); PEREIRA LANGONE, Marta Antunes, CEP: 22630-010 Rio de Janeiro - RJ (BR)
(74) Representative: J A Kemp
(86) International application number: PCT/BR2011/000324
(87) International publication number: WO 2013/037017

(56) References cited:
- WO-A1-96/07751
- WO-A1-2005/014518
- ES-A1- 2 300 197
- JP-A- S6 416 591
- JP-A- H05 211 878
- JP-A- H05 304 966
- BODALO A ET AL: "Production of ricinoleic acid estolide with free and immobilized lipase from Candida rugosa", BIOCHEMICAL ENGINEERING JOURNAL, ELSEVIER, AMSTERDAM, NL, vol. 39, no. 3, 1 May 2008 (2008-05-01), pages 450-456, XP022590976, ISSN: 1369-703X, DOI: 10.1016/J.BEJ.2007.10.013 [retrieved on 2007-10-30]
- TERRY A. ISBELL: "Chemistry and physical properties of estolides", GRASAS Y ACEITES, vol. 62, no. 1, 16 February 2011 (2011-02-16), pages 8-20, XP055153433, ISSN: 0017-3495, DOI: 10.3989/gya/010810
- YOSHIDA, Y ET AL.: 'Enzymatic synthesis by a bioreactor' JAOCS vol. 74, no. 2, 1997, pages 261 - 267, XP055146831
- BODALO A ET AL.: 'Influence of the operating conditions on lipase- catalysed synthesis of ricinoleic acid estolides in solvent-free systems' BIOCHEMICAL ENGENEERING JOURNAL vol. 44, no. 2-3, 2009, pages 214 - 219, XP026006762
- CABRERA Z ET AL: "Novozym 435 displays very different selectivity compared to lipase from Candida antarctica B adsorbed on other hydrophobic supports", JOURNAL OF MOLECULAR CATALYSIS. B, ENZYMATIC, ELSEVIER, AMSTERDAM, NL, vol. 57, no. 1-4, 1 May 2009 (2009-05-01), pages 171-176, XP025991192, ISSN: 1381-1177, DOI: 10.1016/J.MOLCATB.2008.08.012 [retrieved on 2008-08-30]

## Description

### FIELD OF THE INVENTION

This invention falls within the field of enzymatic processes for the synthesis of estolides to be used as lubricants. More specifically, this invention is a process for enzymatic synthesis of estolides through the reaction between stearic acid and methyl ricinoleate, in solvent free media using a specific type of immobilized lipase as catalyst.

### BACKGROUND OF THE INVENTION

Base oils are the principal constituents in the formulation of lubricating oils and are classified as mineral or synthetic. Mineral oils are obtained by distilling and refining petroleum, whereas synthetics are produced by chemical reactions using various raw materials, in an effort to obtain products with properties suitable to the function of lubricants. Mixtures of base oils and additives are normally used to adjust some properties such as viscosity and stability to oxidation and to prevent the formation of deposits.

The imminent depletion of world oil reserves and growing awareness of the environmental impact of its use have stimulated the development of research to obtain fuels and lubricants derived from alternative sources such as vegetable oils. The main advantages of replacing mineral oils with lubricants obtained from vegetable oils are biodegradability and reduced environmental impact. However, such oils are sensitive to thermal-oxidative stress and do not exhibit good performance at low temperatures. The lubricating properties of such compounds can be achieved by modifying the carbon chain of the fatty acids comprising the vegetable oils.

Estolides constitute a new class of lubricants derived from vegetable oils with excellent low temperature properties, the pour point being one of the best indicators of such properties. Estolides is the generic name used to define linear oligomers of polyesters of fatty acids in which the carboxyl group of a fatty acid binds to the unsaturation site of another fatty acid or in which the hydroxyl of a hydroxylated fatty acid is esterified by the carboxyl group of another fatty acid molecule. These lubricants have been synthesized by polymerizing fatty acids under high temperatures or using mineral acid catalysis.

US patent 6,316,649 describes a process for synthesizing esters of estolides through the reaction between oleic acid and saturated fatty acids with 6 to 18 carbon atoms and subsequent reaction with 2-ethylhexanol. Perchloric acid was used as the catalyst, with a reaction temperature ranging from 45° C to 60 ° C. The esters of the estolides obtained exhibited good oxidation stability, a high viscosity index, viscosity compatible with mineral base oils, as well as a low pour point and good degradability.

U.S. patent 6,018,063 refers to a family of estolides derived from oleic acid, produced by acid catalysis, and characterized by its superior properties when used as lubricants, such as viscosity (at 40 °C) between 20 cSt and 32 cSt, viscosity index of 150, and pour point of between-30°C and -21°C, among others. When the 12-hydroxy-stearic acid is used for the production of such esters, there is an increase in the pour point of estolides produced.

One of the disadvantages of the aforementioned processes is that their products must be re-distilled to remove impurities generated by thermal degradation. In addition, the use of strong acids can cause corrosion in industrial equipment and acid effluents that are subsequently difficult to treat.

JP patent 1016591 describes the synthesis of estolides by means of the hydrolysis of hydroxylated fatty acids present in castor oil, more specifically the ricinoleic acid. The hydrolysis reaction and formation of estolide from castor oil was done using 2% to 15% (m/m) of lipase in 30% to 65% (m/m) of castor oil dispersed in an aqueous solution to obtain estolide conversion greater than 90%. The lipases used are those capable of hydrolyzing β positions of the fatty acids or a lipase capable of partially hydrolyzing glycerides.

JP patent 5211878 describes a procedure for obtaining high reaction rates and a high degree of polymerization of estolides, which are free of dark color, odor and impurities. The estolides were synthesized through ricinoleic acid condensation reactions, using immobilized lipase as the catalyst, by controlling the amount of water in the reaction medium. In this case, condensation of ricinoleic acid molecules has the disadvantage of resulting in a hygroscopic product. Moreover, as it is difficult to control the size of the chains of the products obtained, the estolides, it is thus also difficult to maintain the properties of the product for each production.

Bodalo et al, in Biochemical Engineering Journal, vol. 39, no. 3, pages 450-456, refer to the synthesis of estolides from ricinoleic acid catalyzed by immobilized lipase in a solvent-free reaction. Similar processes are described by Yoshida et al, in JAOCS, vol. 74, no. 2, pages 261-267, and by Bodalo et al, in Biochemical Engineering Journal, vol. 44, no. 2-3, pages 214-219.

This invention provides a method for synthesizing estolides through a catalyzed esterification reaction using specific lipases immobilized on macroporous acrylic resins in a solvent-free system as detailed in claim 1. The synthesis of estolides using lipases prevents the degradation of reagents and products, and reduces secondary reactions given that these enzymes act in mild reaction conditions and have high specificity. In addition, the use of immobilized lipases permits their later reuse. Moreover, in a solvent-free system, the purification processes are simpler, since fewer components are present in the reaction medium at the end of the reaction. The elimination of solvents in the production of estolides considerably reduces costs and minimizes environmental impact.

### SUMMARY OF THE INVENTION

This invention is a process for enzymatic synthesis of estolides, comprising the reaction between stearic acid and methyl ricinoleate in a solvent-free medium, using an immobilized lipase as the catalyst,
wherein the lipase, which is non-specific for the 1,3-positions of a triglyceride, is a recombinant lipase from *Candida antarctica*, expressed in *Apergillus Niger,* and is immobilized on macroporous acrylic resins.

### DESCRIPTION OF THE INVENTION

This invention is an enzymatic process for synthesizing estolides in a solvent-free medium, the objective of which is to selectively produce lubricants generating a product with a high degree of purity and reduced waste production. The solvent-free system (SLS) combines the specificity of biological catalysis with reduced operating costs due to lower energy consumption during the reaction and treatment of effluents.

Thus, this invention is a process for synthesizing estolides, via enzymatic catalysis, in a solvent-free medium using an immobilized lipase as the catalyst, wherein the lipase, which is non-specific for the 1,3-positions of a triglyceride, is a recombinant lipase from *Candida antarctica*, expressed in *Apergillus Niger,* and is immobilized on macroporous acrylic resins, with said process including the following steps:
a) Prepare a mixture of stearic acid and methyl ricinoleate, at a molar ratio between 2:1 and 1:1, with agitation and at room temperature;
b) Add the mixture to a reactor containing immobilized lipases in a proportion between 6% and 14% (m/m) lipase in relation to the total concentration of reagents, with agitation and reflux, at temperatures between 70°C and 90°C, for a period of 24 to 100 hours, maintaining water concentration in the reaction medium at less than 0.05% by weight to ensure the expression of the catalytic activity of lipases;
c) Recover the lipase, by removing it from the reaction medium by filtration, obtaining a stream containing estolides at concentrations between 35% and 25% (m/m), which corresponds to a conversion exceeding 40%.

This process utilizes stearic acid from natural sources, particularly vegetable oils and animal fats.

Vegetable oil sources include: cotton seed, coconut, palm, castor, rapeseed, soybean, sunflower seed, and olive oils. Particular mention should be made of cocoa butter and shea butter, which have a high stearic acid content, ranging from 28% to 45%.

In terms of animal fat, sources include milk fat (5% to 15% stearic acid), pig fat (approximately 10% stearic acid), and bovine tallow with 15% to 30% stearic acid.

The sources of methyl ricinoleate (ricinoleic acid ester) useful for this invention are the byproducts of acid transesterification and esterification of castor oil (castor bean biodiesel), as castor oil is roughly 90% made up of ricinoleic acid.

The reaction between stearic acid and methyl ricinoleate is an esterification / condensation reaction, the efficiency of the reaction being dependent on the type and concentration of lipase used, the temperature of the reaction medium and the amount of water in the medium.

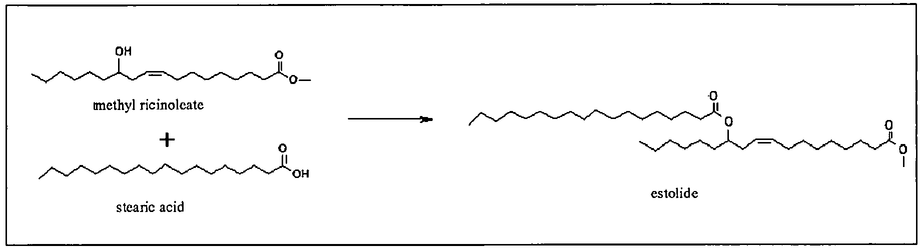

With respect to the type of lipase, it is recognized that lipases with specificity for hydrolysis of the 1, 3-positions of the triglyceride molecules do not act on the hydroxyl group of hydroxylated fatty acids, which are inefficient in the synthesis of estolides derived from these acids. On the other hand, non-specific lipases for the 1, 3-positions are effective in catalyzing this type of reaction. Lipases obtained from genetically-modified microorganisms are used. The lipase is a recombinant lipase from *Candida antarctica*, expressed in *Aspergillus Niger.* The lipase marketed by the Novozymes company as Novozyme 435 can be used.

Thus, the immobilized lipases useful for the process of this invention are not specific for the 1, 3-positions, and are immobilized on macroporous acrylic resins, especially in concentrations greater than 10,000 U/g.

Apart from selecting the lipase, another variable to be observed in the enzymatic synthesis of estolides is the influence of temperature on the enzymatic reaction, as a result of decreased enzyme stability due to thermal deactivation. The ideal working temperature for lipase varies from 70°C to 90°C. At temperatures below 70°C, low consumption of fatty acid molecules is observed, the conversion to estolides being less than 30%.

The lipase concentration suitable for the process ranges from 6% to 14% (m/m) relative to the total concentration of reagents. In lipase concentrations below 6% (m/m), the conversion is low, generally below 30%. Enzyme concentrations above 14% (m/m) contain an agglomeration of particles of immobilized lipase, reducing the active sites available for reaction, causing a reduction in yield and conversion.

The esterification reaction generates water as a byproduct; however, its presence in the reaction medium adversely affects conversion rates, since when the water content in the reaction mixture increases, the reaction reaches an equilibrium and stops. Therefore, the removal of water produced during the reaction is extremely important for the conversion to be high, above 40%.

The removal of water from the reaction medium can be done by means of an adsorbent in concentrations between 4% and 7% (m/m). The following may be cited among the adsorbents useful for this invention: alumina, silica gel, zeolites, preferably molecular sieves, or the application of a 60 Pa (0.6 mbar) vacuum.

Thus, this invention is an enzymatic process, the objective of which is the synthesis of estolides from the reaction between stearic acid and methyl ricinoleate, using immobilized lipases as the catalyst.

Due to the use of lower temperatures in enzymatic catalysis, compared to those of acid catalysis (temperatures between 205°C and 210°C), it is possible to avoid degrading the product, which, in estolides, is caused by the oxidation of fatty acids. Furthermore, it is also possible to reduce the formation of secondary products that generate color and odor in the lubricant by using highly specific lipases as the catalyst and it is also possible to recover the lipases for their later reuse and/or recycling.

In this case, the lipase can be recovered by vacuum filtration, using n-hexane as a solvent.

Therefore, the use of methyl ricinoleate in condensation reactions with stearic acid, catalyzed by lipases, creates a biodegradable lubricant of high added value, which expands the application scope of castor oil biodiesel and can lead to its commercially viable production.

The following examples correspond to experiments conducted on a laboratory scale, which are merely illustrative of the synthesis process described in detail up to this point, and, therefore, do not limit its scope.

### SPECIFIC EXAMPLES OF THE INVENTION:

### EXAMPLE 1

284.5 g of commercial stearic acid (99.0% stearic acid) and 312.5 g methyl ricinoleate (obtained through the transesterification of castor oil) were added to a batch reactor containing 60 g of commercial immobilized lipase (Novozyme 435). The average reaction temperature was maintained at 84°C for 24 hours, with constant agitation of 100 rpm. The product (estolides) was recovered, and 43% reagent conversion was achieved.

### EXAMPLE 2

853.5 g of stearic acid (P.A. grade) and 937.5 g of methyl ricinoleate (obtained through the transesterification of castor oil) were added to a batch reactor loaded with commercial immobilized lipase (Novozyme 435), such that the enzyme concentration in the reactants was 9% (m/m). The average reaction temperature was kept at 85°C for 100 hours, with agitation, and 50% reagent conversion was achieved.

### EXAMPLE 3

The following example illustrates the effect of the presence of water in the reaction medium in the conversion of the reaction, through the action of an agent for removing water, in this case a molecular sieve.

853.5 g of stearic acid (P.A. purity grade) were added to a batch reactor loaded with commercial immobilized lipase (Novozyme 435), as well as 937.5 g of methyl ricinoleate (obtained through the transesterification of castor oil) and 500 mg from a 3A molecular sieve, so that the concentration of enzyme to the reactants was 9% (m/m). The temperature was kept at 85°C for 48 hours, and 51% conversion was achieved.

### EXAMPLE 4

The following example illustrates the effect of the presence of water in the reaction medium on the conversion of the reaction by removal of water through application of a vacuum.

1.70 kg of stearic acid and 1.87 kg of methyl ricinoleate (obtained through the transesterification of castor oil) were added to a batch reactor loaded with commercial immobilized lipase (Novozyme 435), such that the enzyme concentration relative to the reactants was 10% (m/m). The temperature was kept at 84°C for 24 hours under vacuum and conversion reached 44%

### EXAMPLE 5

### Reuse of the lipase

The possibility of reusing commercial lipase in reactions between stearic acid and methyl ricinoleate is illustrated in Table 1, which shows that the enzyme may be reused in four consecutive reactions.

| TABLE 1 | | | | |
|---|---|---|---|---|
| BATCH | 1 | 2 | 3 | 4 |
| CONVERSION | 33 | 28 | 20 | 15 |

### EXAMPLE 6

### Characterization of the biolubrificant

The properties of the final product: viscosity, viscosity index, pour point and corrosiveness, were evaluated to characterize the biolubricant obtained. The biolubricant obtained showed good viscosity properties, a low pour point and an absence of corrosiveness, as compared to a base oil and a commercial lubricant (Lubrax Unitractor), as shown in Table 2.

| TABLE 2 | | | | | |
|---|---|---|---|---|---|
| Lubricant | Pour point³ | Viscosity at 40°C⁴ (cSt) | Viscosity at 100°C⁵ (CSt) | Index of viscosity⁶ | Corrosion on a copper strip⁷ |
| Estolide¹ | -24°C | 23.9 cSt | 5.2 cSt | 153 | 1A |
| Spindle 09² | -27 | 10.7 | 2.7 | 95 | - |
| Lubrax Unitractor | -39 | 54 | 9.3 | 156 | 1B |

| | | | | | |
|---|---|---|---|---|---|
| 1- Produced following the procedure described in this invention. 2- Mineral oil. 3- Pour point calculated using the ASTM D97/07 method. 4- Viscosity at 40°C calculated using the ASTM D445/09 method. 5- Viscosity at 100°C calculated using the ASTM D445/09 method. 6- Viscosity index calculated using the ASTM D2270/04 method. 7- Corrosion on a copper strip calculated using the ASTM D130/04 method. | | | | | |

## Claims

1. Process for the enzymatic synthesis of estolides, comprising the reaction of stearic acid and methyl ricinoleate in a solvent-free medium, using an immobilized lipase as the catalyst,
wherein the lipase, which is non-specific for the 1,3-positions of a triglyceride, is a recombinant lipase from *Candida antarctica*, expressed in *Apergillus Niger*, and is immobilized on macroporous acrylic resins.

2. A process according to claim 1, comprising the following steps:
a) preparing a mixture of stearic acid and methyl ricinoleate, at a molar ratio between 2:1 and 1:1, with agitation and at room temperature;
b) adding the mixture to a reactor containing the immobilized lipase, in a ratio between 6% and 14% (m/m) lipase in relation to the total concentration of reagents, with agitation and reflux, at temperatures between 70°C and 90°C, for a period of 24 to 100 hours, maintaining the water concentration in the reaction medium at less than 0.05% by weight to ensure the expression of the catalytic activity of the lipase; and
c) recovering the immobilized lipase, by removing it from the reaction medium through filtration, obtaining a stream containing estolides at concentrations between 35% and 25% (m/m).

3. A process according to claim 1 or 2, wherein the lipase is immobilized on macroporous acrylic resins, in concentrations greater than 10,000 U/g.

4. A process according to claim 1 or 2, wherein the stearic acid has an oil of vegetable origin as its source.

5. A process according to claim 4, wherein the oil of vegetable origin is selected from among: cottonseed, coconut, palm, castor bean, rapeseed, soybean, sunflower seed and olive oil.

6. A process according to claim 1 or 2, wherein the stearic acid has an animal fat as its source.

7. A process according to claim 6, wherein the animal fat is selected from among: milk fat, pork fat and bovine tallow.

8. A process according to claim 1 or 2, wherein the concentration of water in the reaction medium is maintained using the addition of adsorbents to the reaction medium in the proportion of 4% and 7% (m/m).

9. A process according to claim 8, wherein the adsorbent is selected from: alumina, silica gel, zeolites.

10. A process according to claim 8, wherein the adsorbent is a molecular sieve.

11. A process according to claim 1 or 2, wherein the water in the reaction medium is maintained by applying a vacuum at a pressure of 60 Pa.

12. A process according to claim 1 or 2, wherein the lipase is recovered by vacuum filtration.

13. A process according to claim 1, 2 or 12, wherein the recovered lipase enters into contact with a solvent that is n-hexane.

## Patentansprüche

1. Verfahren zur enzymatischen Synthese von Estoliden, umfassend die Reaktion von Stearinsäure und Methylricinoleat in einem lösungsmittelfreien Medium unter Verwendung einer immobilisierten Lipase als Katalysator,
wobei die Lipase, die nicht spezifisch für die 1,3-Positionen eines Triglycerids ist, eine rekombinante Lipase aus *Candida antarctica*, exprimiert in *Apergillus Niger*, ist und auf makroporösen Acrylharzen immobilisiert ist.

2. Verfahren nach Anspruch 1, umfassend die folgenden Schritte:
a) Herstellen eines Gemischs aus Stearinsäure und Methylricinoleat in einem Molverhältnis zwischen 2:1 und 1:1 mit Rühren und bei Raumtemperatur;
b) Hinzufügen des Gemischs zu einem Reaktor, der die immobilisierte Lipase enthält, in einem Verhältnis zwischen 6 % und 14 % (m/m) Lipase bezogen auf die Gesamtkonzentration von Reagenzien mit Rühren und Reflux bei Temperaturen zwischen 70 °C und 90 °C über einen Zeitraum von 24 bis 100 Stunden, wobei die Wasserkonzentration in dem Reaktionsmedium auf weniger als 0,05 Gew.-% gehalten wird, um die Expression der katalytischen Aktivität der Lipase zu gewährleisten; und
c) Gewinnen der immobilisierten Lipase, indem sie durch Filtration aus dem Reaktionsmedium entfernt wird, wodurch ein Strom erlangt wird, der Estolide in Konzentrationen zwischen 35 % und 25 % (m/m) enthält.

3. Verfahren nach Anspruch 1 oder 2, wobei die Lipase auf makroporösen Acrylharzen in Konzentrationen größer als 10.000 U/g immobilisiert ist.

4. Verfahren nach Anspruch 1 oder 2, wobei die Stearinsäure ein Öl pflanzlichen Ursprungs als seine Quelle aufweist.

5. Verfahren nach Anspruch 4, wobei das Öl pflanzlichen Ursprungs aus Folgenden ausgewählt ist: Baumwollsamen-, Kokosnuss-, Palm-, Rizinussamen-, Raps-, Sojabohnen-, Sonnenblumenkern- und Olivenöl.

6. Verfahren nach Anspruch 1 oder 2, wobei die Stearinsäure ein tierisches Fett als seine Quelle aufweist.

7. Verfahren nach Anspruch 6, wobei das tierische Fett aus Folgenden ausgewählt ist: Milchfett, Schweinefett und Rindertalg.

8. Verfahren nach Anspruch 1 oder 2, wobei die Konzentration von Wasser in dem Reaktionsmedium unter Verwendung des Hinzufügens von Adsorbtionsmitteln zu dem Reaktionsmedium in dem Anteil von 4 % und 7 % (m/m) aufrechterhalten wird.

9. Verfahren nach Anspruch 8, wobei das Adsorptionsmittel aus Folgenden ausgewählt ist: Aluminiumoxid, Kieselgel, Zeolithen.

10. Verfahren nach Anspruch 8, wobei das Adsorptionsmittel ein Molekularsieb ist.

11. Verfahren nach Anspruch 1 oder 2, wobei das Wasser in dem Reaktionsmedium durch Anwenden eines Vakuums bei einem Druck von 60 Pa beibehalten wird.

12. Verfahren nach Anspruch 1 oder 2, wobei die Lipase durch Vakuumfiltration gewonnen wird.

13. Verfahren nach Anspruch 1, 2 oder 12, wobei die gewonnene Lipase in Kontakt mit einem Lösungsmittel kommt, das n-Hexan ist.

## Revendications

1. Procédé de synthèse enzymatique d'estolides, comprenant la réaction d'acide stéarique et de ricinoléate de méthyle dans un milieu sans solvant, au moyen d'une lipase immobilisée comme catalyseur,
dans lequel la lipase, qui n'est pas spécifique aux positions 1 à 3 d'un triglycéride, est une lipase recombinante issue de *Candida antarctica*, exprimée dans de l'*Apergillus Niger*, et est immobilisée sur des résines acryliques macroporeuses.

2. Procédé selon la revendication 1, comprenant les étapes suivantes :
a) la préparation d'un mélange à base d'acide stéarique et de ricinoléate de méthyle, dans un rapport molaire compris entre 2:1 et 1:1, avec agitation et à température ambiante ;
b) l'ajout du mélange dans un réacteur contenant la lipase immobilisée, avec un rapport entre 6 % et 14 % (m/m) de lipase par rapport à la concentration totale des réactifs, avec agitation et reflux, à des températures comprises entre 70 °C et 90 °C pendant une période allant de 24 à 100 heures, maintenant la concentration d'eau dans le milieu réactionnel inférieure à 0,05 % en poids pour garantir l'expression de l'activité catalytique de la lipase ; et
c) la récupération de la lipase immobilisée, en la retirant du milieu réactionnel via une filtration, pour obtenir un courant contenant des estolides à des concentrations comprises entre 35 % et 25 % (m/m).

3. Procédé selon la revendication 1 ou 2, dans lequel la lipase est immobilisée sur des résines acryliques macroporeuses, dans des concentrations supérieures à 10 000 U/g.

4. Procédé selon la revendication 1 ou 2, dans lequel l'acide stéarique comporte une huile d'origine végétale comme source.

5. Procédé selon la revendication 4, dans lequel l'huile d'origine végétale est choisie parmi : l'huile de coton, de coco, de palme, de ricin, de colza, de soja, de tournesol et l'huile d'olive.

6. Procédé selon la revendication 1 ou 2, dans lequel l'acide stéarique comporte une graisse animale comme source.

7. Procédé selon la revendication 6, dans lequel la graisse animale est choisie parmi : la matière grasse laitière, le gras de porc et le suif d'origine bovine.

8. Procédé selon la revendication 1 ou 2, dans lequel la concentration d'eau dans le milieu réactif est maintenue en ajoutant des absorbants au moyen réactionnel dans la proportion de 4 % et 7 % (m/m).

9. Procédé selon la revendication 8, dans lequel l'absorbant est choisi parmi : l'alumine, le gel de silice, les zéolites.

10. Procédé selon la revendication 8, dans lequel l'absorbant est un tamis moléculaire.

11. Procédé selon la revendication 1 ou 2, dans lequel l'eau dans le milieu réactionnel est maintenue en appliquant un vide à une pression de 60 Pa.

12. Procédé selon la revendication 1 ou 2, dans lequel la lipase est récupérée par une filtration sous vide.

13. Procédé selon la revendication 1, 2 ou 12, dans lequel la lipase récupérée entre en contact avec un solvant qui est un n-hexane.
